**Europäisches Patentamt**

**European Patent Office**

**Office européen des brevets**

(19)

(11) Numéro de publication : **0 366 535 B1**

# FASCICULE DE BREVET EUROPEEN

(12)

(45) Date de publication du fascicule du brevet :
**30.12.92 Bulletin 92/53**

(51) Int. Cl.$^5$ : **A61M 29/02, A61M 5/172**

(21) Numéro de dépôt : **89402919.8**

(22) Date de dépôt : **24.10.89**

(54) **Appareillage pour la réalisation d'une angioplastie prolongée.**

(30) Priorité : **27.10.88 FR 8814022**

(43) Date de publication de la demande :
**02.05.90 Bulletin 90/18**

(45) Mention de la délivrance du brevet :
**30.12.92 Bulletin 92/53**

(84) Etats contractants désignés :
**BE CH DE ES GB IT LI NL SE**

(56) Documents cités :
**EP-A- 0 192 786
EP-A- 0 246 998
WO-A-88/00071
US-A- 4 392 847**

(73) Titulaire : **Farcot, Jean-Christian
20, rue Parmentier
F-92200 Neuilly-sur Seine (FR)**

(72) Inventeur : **Farcot, Jean-Christian
20, rue Parmentier
F-92200 Neuilly-sur Seine (FR)**

(74) Mandataire : **Boutin, Antoine
Cabinet Tony-Durand, 77, Rue Boissière
F-75116 Paris (FR)**

## Description

La présente invention concerne le matériel utilisé pour la réalisation d'une angioplastie dans une artère coronaire ou une artère périphérique.

Une telle opération est actuellement pratiquée en utilisant un cathéter constitué par un tube enfilé sur une tige flexible de guidage et dont l'extrémité distale porte un ballonnet de dilatation susceptible d'être gonflé par insufflation d'air dans ce tube. Grâce à la tige de guidage il est possible de placer le ballonnet gonflable à l'endroit précis où il convient de procéder à une dilatation afin d'écraser des plaques athéromateuses de façon à rétablir un passage suffisant de circulation. Cependant cette technique a pour inconvénient que pendant toute la durée de gonflage du ballonnet de dilatation, la circulation sanguine est complètement interrompue à l'intérieur de l'artère correspondante. Dans le cas d'une artère coronaire ceci peut entraîner une ischémie des muscles cardiaques pouvant provoquer divers phénomènes nuisibles ou dangereux tels que : modifications électriques, douleurs thoraciques, anomalies de la contractilité ventriculaire gauche. Du reste ceci peut conduire à des arythmies pouvant mettre la vie du malade en danger.

C'est la raison pour laquelle les angioplasties ne peuvent être pratiquées que pendant un temps relativement court, de l'ordre de 1 à 3 mn. Dans ces conditions, une angioplastie doit être répétée plusieurs fois, ce qui expose le malade aux risques mentionnés ci-dessus. Par ailleurs les angioplasties présentent un taux de resténose de l'ordre de 30% à six mois, ce qui oblige à de nouvelles opérations de dilatation. Cependant il résulte des publications médicales en la matière que le taux de resténose est inversement proportionnel à la durée de l'angioplastie pratiquée.

Il est donc nécessaire de développer des techniques permettant d'augmenter le temps d'inflation des ballonnets standards de dilatation. A cet effet il a déjà été proposé dans le WO 88/00071 d'utiliser un ballonnet gonflable de type particulier permettant la continuation de la circulation normale du sang à travers ce ballonnet pendant son gonflement. A cet effet, ce ballonnet comporte deux parois concentriques et la paroi intérieure délimite un espace ouvert à ses deux bouts de façon à permettre le libre passage de la circulation sanguine. Le volume gonflable de ce ballonnet est alors constitué par l'espace annulaire existant entre les deux parois concentriques et qui est fermé à ses deux extrémités, le tube d'insufflation débouchant à l'intérieur de cet espace.

Cependant l'expérience prouve que cette technique n'est pas suffisante pour assurer une parfaite irrigation des muscles cardiaques pendant un traitement d'angioplastie.

C'est pourquoi la présente invention a pour objet un appareillage conçu de façon à pouvoir réaliser une technique différente permettant une réelle prolongation de la durée d'une angioplastie, et ce sans aucun risque pour le malade à traiter.

A cet effet, cet appareillage est caractérisé en ce que :

- le ballonnet gonflable est traversé sur toute sa longueur par un tube débouchant au delà de celui-ci et qui s'étend sur toute la longueur de la conduite d'insufflation d'air, ce tube étant destiné à assurer une perfusion de sang, ou de liquide physiologique, au delà du ballonnet de dilatation lors de son gonflement,

- l'extrémité de ce tube, qui est opposée à ce ballonnet est raccordée à une pompe de perfusion dont le fonctionnement est asservi à un appareil de commande susceptible d'assurer la synchronisation de son fonctionement à l'électrocardiogramme du malade à traiter afin d'assurer, en avant du ballonnet de dilatation, une perfusion de sang, ou de liquide physiologique transporteur d'oxygène, suivant un débit variable correspondant aux phases des diastoles et des systoles physiologiques du rythme cardiaque de ce malade,

- la tige de guidage étant montée sur le tube de perfusion, ou sur le ballonnet gonflable, de façon à ne pas entraver la circulation de liquide à travers ce tube et à la sortie de celui-ci.

Dans ces conditions, le présent appareillage permet de réaliser, au delà du ballonnet de dilatation, une perfusion réalisant une circulation "forcée" de sang dont le débit variable correspond très exactement aux phases du rythme cardiaques du malade traité. Or les expériences réalisées ont permis de constater que dans le cas d'une artère coronaire, une telle perfusion permet d'éviter complètement les inconvénients et les risques exposés précédemment car le muscle cardiaque est irrigué dans des conditions proches de la normale par le sang ou par un liquide physiologique transporteur d'oxygène. En conséquence la durée d'une angioplastie peut être prolongée de façon importante avec tous les avantages que cela comporte.

Dans une forme de réalisation avantageuse du présent appareillage, l'appareil de commande de la pompe de perfusion comporte plusieurs dispositifs distincts de commande aptes à faire fonctionner cette pompe suivant les caractéristiques particulières de la courbe de débit, pendant les phases de diastoles et de systoles, qui est propre à l'une ou l'autre des artères susceptibles d'être traitées -par exemple : artère interventriculaire antérieure (IVA), artère circomflexe, artère coronaire droite, artères périphériques-, des organes de commande permettant de mettre en service l'un ou l'autre de ces dispositifs suivant le cas, et l'agencement de l'appareil de commande étant tel que le fonctionnement du dispositif mis en service est asservi à l'électrocardiogramme du malade, en ce qui concerne le moment de départ des phases de diastoles et de systoles.

Dans ces conditions, la perfusion réalisée par la pompe est modifiée, en débit et en pression, pour correspondre très exactement aux conditions normales de circulation sanguine à l'intérieur de l'artère traitée, et ce aussi bien dans le cas d'une artère coronaire que dans celui d'une artère périphérique.

Pour permettre la perfusion de sang, ou de liquide physiologique transporteur d'oxygène, à travers un passage de section maximum à l'intérieur d'un cathéter de type standard, la tige de guidage est montée sur ou dans celui-ci de façon à ne pas obstruer son passage intérieur. A cet effet, le cathéter utilisé et sa tige de guidage sont réalisés et associés l'un à l'autre de façon particulière. Dans les diverses formes de réalisation prévues, l'agencement est tel que celui-ci évite un effet de jet du liquide perfusé, à la sortie de l'orifice terminal du cathéter. A cet effet, cette extrémité comporte avantageusement des orifices répartis sur sa périphérie et qui permettent une sortie du liquide perfusé tout autour de cette extrémité, et ce de façon préférentielle par rapport à la sortie à travers l'orifice terminal.

Cependant d'autres particularités et avantages de l'appareillage selon l'invention apparaitront au cours de la description suivante. Celle-ci est donnée en référence aux dessins annexés à simple titre indicatif, et sur lesquels :

La figure 1 est une vue schématique d'ensemble illustrant le principe de l'appareillage selon l'invention, celui-ci étant branché d'une part sur une artère coronaire à dilater, et d'autre part sur un vaisseau d'alimentation constitué soit par une artère périphérique, soit par une veine appartenant à, ou provenant d'un système veineux très oxygéné.

La figure 2 est un schéma du circuit des différents dispositifs de commande prévus dans cet appareillage.

La figure 3 est une vue schématique du cathéter et de son ballonnet de dilatation.

Les figures 4 à 7 sont des vues en élévation de quatre formes de réalisation différentes de l'extrémité distale du cathéter.

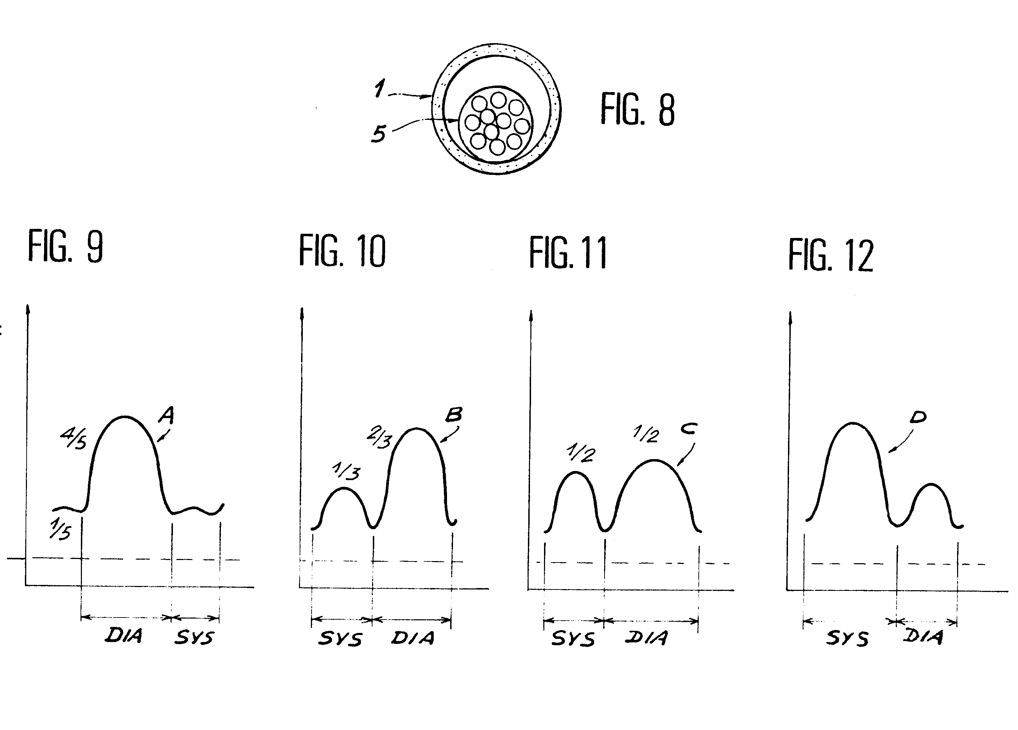

La figure 8 est une vue en coupe selon la ligne VIII-VIII de la figure 7.

Les figures 9 à 12 représentent les courbes caractéristiques de la circulation sanguine dans différentes artères susceptibles d'être traitées avec le présent appareillage.

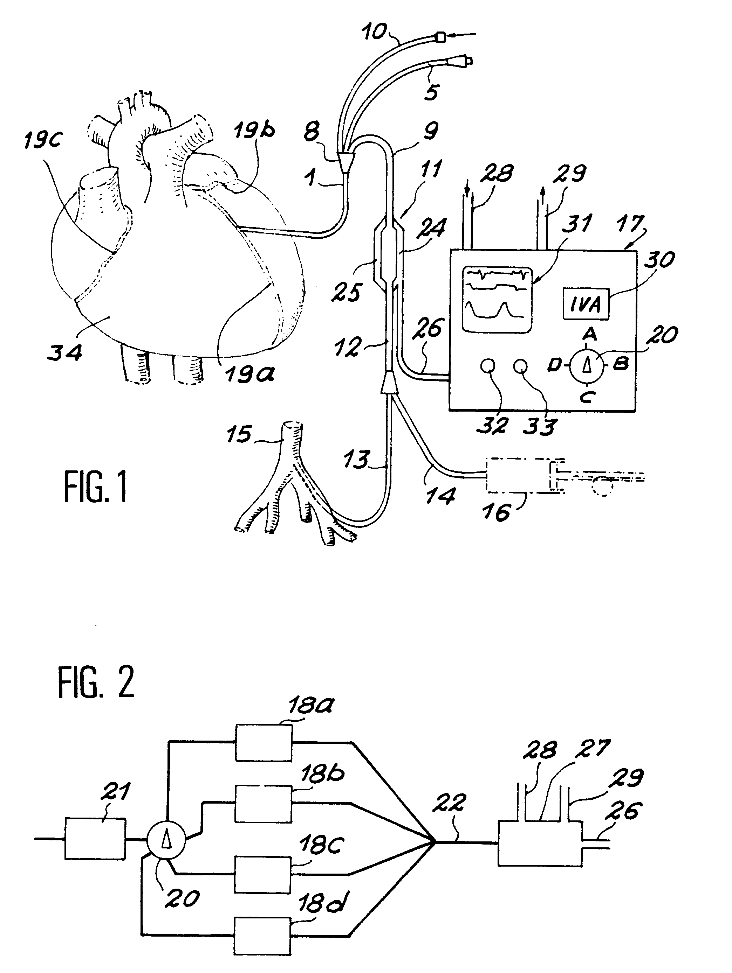

L'appareillage représenté aux figures 1 à 3 comprend un cathéter constitué par un ballonnet gonflable 3 de dilatation, prévu au bout d'une conduite 4 d'insufflation d'air. Cette conduite est solidaire d'un tube 1 de même longueur dont l'extrémité distale 2 traverse le ballonnet 3 sur toute sa longueur pour déboucher au delà de celui-ci. Comme il sera décrit plus en détail par la suite, ce tube 1 est destiné à assurer, à l'intérieur de l'artère traitée par angioplastie, une perfusion de sang au delà du ballonnet 1 pendant le gonflement de celui-ci.

Le tube 1 de perfusion est enfilé sur une tige flexible de guidage 5, constituée par exemple par un câble métallique tressé ou un fil métallique enroulé en hélice. Cette tige de guidage 5 présente une section de surface nettement inférieure à celle de la section du tube de perfusion, par exemple une surface équivalente à la moitié ou au tiers de celle de ce tube, afin de ne pas obstruer l'espace intérieur de celui-ci et de laisser un passage libre pour la perfusion de sang ou de liquide physiologique porteur d'oxygène, et ce au-delà du ballonnet de dilatation. Cependant, l'extrémité 6 de cette tige de guidage présente pour sa part une section accrue d'importance normale, mais ce nettement au-delà de l'extrémité 2 du tube 1 de perfusion afin de laisser un passage de sortie à l'endroit de l'orifice terminal 7 de celui-ci. Il convient de noter que cette section accrue est néanmoins inférieure à celle du canal du tube 1 de perfusion pour permettre le coulissement de la tige de guidage à l'intérieur de celui-ci.

A son extrémité opposée le tube 1 de perfusion porte un embout 8 au-delà duquel s'étend l'extrémité correspondante de la tige de guidage 5. Sur cet embout sont branchées deux conduites 9 et 10 communiquant respectivement avec le tube 1 et avec la conduite 4 de gonflage du ballonnet 3 de dilatation.

La conduite 9 est destinée à assurer l'acheminement du liquide de perfusion dans le tube 1. A cet effet, elle est branchée sur une unité pulsatile 11 destinée à servir de pompe de perfusion. Sur l'orifice d'entrée de cette pompe est branchée une conduite 12 d'alimentation qui se subdivise en amont en deux canalisations 13 et 14. La première d'entre elles est constituée par un cathéter susceptible d'être introduit dans une artère périphérique 15 du malade traité ou bien dans une veine d'un système veineux très oxygéné (abouchement des artères rénales) de celui-ci. Cette canalisation 13 est ainsi en mesure d'alimenter la pompe 11 en sang très oxygéné. Quant à la canalisation 14, elle est branchée sur une pompe de refoulement 16 permettant l'injection de médicament ou bien éventuellement une alimentation en un liquide physiologique transporteur d'oxygène, et ce à la place de sang naturel.

L'unité pulsatile 11 constituant la pompe de perfusion est apte à aspirer le liquide à perfuser, provenant de la canalisation 13 ou 14, et à le refouler périodiquement vers la conduite 9 et le tube 1 de perfusion. A cet effet, le fonctionnement de cette unité pulsatile est asservi à un appareil de commande désigné par la référence générale 17. Celui-ci est conçu de manière que le fonctionnement de cette pompe de perfusion soit synchronisé avec l'électrocardiogramme du malade traité afin que le débit d'injection de liquide de perfusion dans le tube 1 soit assuré de façon variable, et ce en synchronisme avec les phases des diastoles et des systoles du rythme cardiaque du malade traité.

Cependant l'appareil de commande 17 comporte en outre plusieurs dispositifs distincts de commande aptes à faire fonctionner la pompe 11 de perfusion suivant les caractéristiques particulières de la courbe de débit qui est propre à l'une ou l'autre des artères susceptibles d'être traitées. Ainsi, il peut être prévu quatre dispositifs distincts de commande indiqués par les références générales 18a, 18b, 18c et 18d sur le schéma de la figure 2. Le premier de ceux-ci 18a correspond par exemple à la courbe de débit A dans l'artère interventriculaire antérieure 19a (IVA), le second 18b à la courbe B de débit dans l'artère circonflexe 19b, le troisième 18c à la courbe de débit C dans l'artère coronaire droite 19c et enfin le dernier 18d à la courbe D de débit dans les artères périphériques.

Comme représenté sur le schéma de la figure 2, un bouton de commande 20 permet de raccorder l'un ou l'autre de ces dispositifs avec le dispositif général de commande 21 qui est asservi à l'électrocardiogramme du malade. En conséquence, le circuit de commande 22 passe par l'un ou l'autre de ces dispositifs 18a....18d pour aboutir au système 23 d'actionnement de la pompe de perfusion.

Dans l'exemple représenté, cette pompe est constituée par une unité pulsatile à fonctionnement pneumatique, hydraulique ou électromécanique. Dans l'exemple représenté cette unité pulsatile comporte une poche déformable 24 disposée à l'intérieur d'une enceinte 25 et dont l'une des extrémités est raccordée à la conduite d'alimentation 12 cependant que sur l'autre est branchée la conduite 9 de sortie. L'espace intérieur de l'enceinte 25 est raccordé, par une conduite 26, au système d'actionnement, en l'occurrence une électrovanne 27 commandée par le circuit 22. Sur cette électrovanne sont branchées d'une part une conduite 28 d'alimentation en air ou liquide sous pression et d'autre part une conduite d'évacuation 29. Ceci permet de commander successivement la contraction et la dilatation de la poche déformable 24 faisant partie de l'unité pulsatile 11. En conséquence ceci assure l'envoi d'un débit plus ou moins important de liquide de perfusion vers le cathéter 1, et ce en fonction des consignes provenant à la fois du dispositif général de commande 21 asservi au rythme cardiaque du malade traité, et à l'un ou l'autre des dispositifs 18a.....18d correspondant aux caractéristiques propres de débit dans l'artère traitée.

En plus du bouton de commande 20 déjà mentionné, le boîtier de l'appareil de commande 17 comporte un écran 30 sur lequel apparait une indication d'identification de l'artère traitée et qui correspond à la position de ce bouton (par exemple IVA pour l'artère interventriculaire antérieure). Cependant cet appareil comporte un autre écran 31 sur lequel est affiché l'électrocardiogramme du malade traité. Enfin il comporte un bouton 32 de mise en fonctionnement ainsi qu'un ou plusieurs boutons de réglage 33.

L'exemple illustré à la figure 1 correspond à la réalisation d'une angioplastie dans l'artère interventriculaire antérieure 19a du coeur 34 d'un malade. Après introduction du cathéter 1 dans cette artère et mise en place du ballonnet 3 de dilatation à l'endroit où doit être pratiquée une angioplastie, l'appareil de commande 17 est mis en fonctionnement pour assurer une perfusion en avant de ce ballonnet pendant toute l'opération. Bien entendu il convient de régler au préalable cet appareil en fonction de la nature de l'artère traitée. Ainsi dans l'exemple représenté, l'index du bouton 20 est placé en regard du repère A correspondant à la mise en service du dispositif annexe de commande 18a correspondant à l'artère interventriculaire antérieure. De ce fait, la perfusion au-delà du ballonnet de dilatation est réalisée suivant les caractéristiques de la courbe de débit à l'intérieur de l'artère correspondante, et en synchronisme avec le rythme cardiaque du malade traité. En effet, les différents dispositifs annexes de commande 18a.....18d sont asservis au dispositif général de commande 21, lui-même asservi à l'électrocardiogramme du malade. En conséquence le débit de perfusion est variable et les variations de débit correspondent à celles des phases de diastole et de systole, et ce en synchronisme avec celles du rythme cardiaque du malade.

De ce fait l'irrigation du coeur pendant l'angioplastie est réalisée par une circulation "forcée" de sang, mais en synchronisme avec le rythme cardiaque du malade, ce qui permet d'éviter les inconvénients et risques exposés précédemment. Pour cette raison, la durée de l'angioplastie peut être prolongée de façon importante. Ceci permet donc d'augmenter l'efficacité de cette opération en évitant des risques de resténose.

Il convient de noter que l'ensemble formé par les cathéters et autres canalisations employés avec la pompe 11, est un système à usage unique pour chaque malade, ce système étant destiné à être remplacé par un autre après chaque utilisation du présent appareillage. De plus, ce système est stérile et les différentes canalisations véhiculant le sang ne sont jamais en contact avec l'air ou avec l'atmosphère extérieure. Par ailleurs, le présent appareillage est suffisamment puissant pour que l'unité pulsatile puisse délivrer autant de sang, ou autre liquide de perfusion, que nécessaire pour assurer une perfusion physiologique efficace de la zone à irriguer.

Cependant le présent appareillage peut faire l'objet de diverses variantes et autres formes de réalisation.

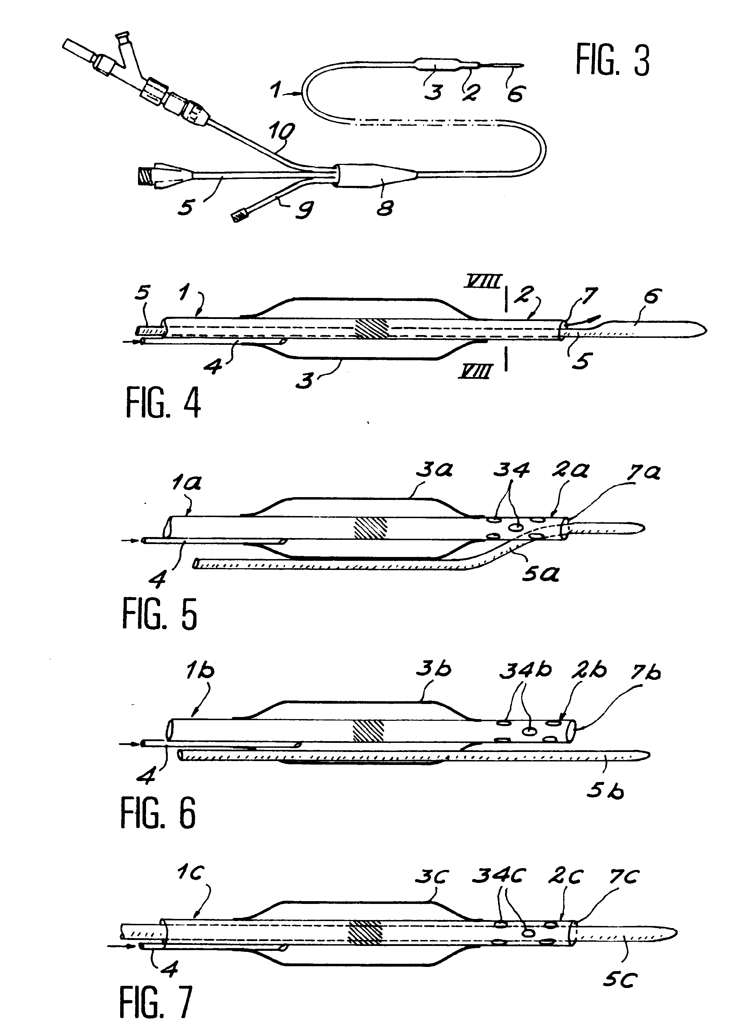

Ainsi, la figure 5 représente une autre forme de réalisation du cathéter faisant partie du présent appareillage. Dans celle-ci, la tige flexible de guidage 4a est disposée en dehors du tube de perfusion correspondant 1a et du ballonnet de dilatation 3a porté par celui-ci. Cependant l'extrémité de cette tige est engagée dans l'extrémité distale 2a du tube de perfusion et sort à travers l'orifice terminal 7a de celui-ci. Dans ces conditions, cet orifice se trouve à peu près complètement obstrué par cette tige de guidage.

C'est la raison pour laquelle l'extrémité 2a du tube de perfusion comporte une série d'orifices 34 destinés à permettre la sortie du liquide de perfusion. Ces orifices se trouvent alors situés au-delà du ballonnet de dilatation 3a, mais en amont du point d'introduction de l'extrémité de la tige de guidage 4a à l'intérieur de l'extrémité du tube de perfusion. Dans ces conditions, celui-ci permet de réaliser, comme précédemment, une perfusion au-delà du ballonnet de dilatation, et ce pendant toute la durée du gonflement de ce dernier. L'existence des orifices 34 permet de briser la force du jet sortant par l'orifice terminal 7a, lorsque la tige de guidage 5a est retirée pendant l'angioplastie.

La figure 6 représente une autre forme de réalisation dans laquelle la tige de guidage 4b est entièrement disposée en dehors du tube de perfusion correspondant 1b. Cependant cette tige de guidage traverse le ballonnet de dilatation 3b porté par ce tube. Ceci assure donc la liaison voulue de cette tige de guidage avec ce dernier.

De même que dans la forme de réalisation représentée à la figure 5, l'extrémité 2b du tube de perfusion 1b comporte une série d'orifices 34b répartis sur sa périphérie et qui sont destinés à permettre la sortie du liquide de perfusion. Dans le cas présent, cette sortie peut également s'effectuer à travers l'orifice terminal 7b du tube de perfusion. Cependant les orifices périphériques 34b sont prévus pour éviter un effet de jet à la sortie de l'orifice 7b. En effet, il convient d'éviter un tel effet qui aurait pour inconvénient de décoller ou de mutiler l'endothélium du vaisseau traité. Dans ce but, la somme de la surface des orifices 34b est supérieure à la section de l'orifice terminal 7b du tube de perfusion 1b. Ainsi, la sortie du liquide de perfusion s'effectue de façon préférentielle à travers les orifices 34b prévus à la périphérie de l'extrémité du cathéter.

La figure 7 représente une autre variante de réalisation encore dans laquelle la tige de guidage 4c est destinée à être retirée hors du tube de perfusion 1c après mise en place du ballonnet de dilatation 3c à l'endroit voulu. Dans cette forme de réalisation, l'extrémité distale 2c du tube de perfusion comporte, de même que dans le cas de la figure 6, une série d'orifices de sortie 34c ménagés à sa périphérie. Là encore la somme des surfaces de ces différents orifices est supérieure à la section de l'orifice terminal 7c du tube de perfusion de façon à éviter un effet de jet lors de la perfusion après retrait de la tige flexible de guidage 4c.

Mais il ne s'agit là que de simples exemples de réalisation, l'appareillage selon l'invention pouvant donner lieu à de nombreuses autres formes de réalisation.

**Revendications**

1. Appareillage pour la réalisation d'une angioplastie prolongée d'une artère coronaire ou d'une artère périphérique, comprenant un cathéter constitué par un ballonnet gonflable de dilatation fixé en bout d'une conduite d'insufflation d'air associée à une tige flexible de guidage, caractérisé en ce que :

   - le ballonnet gonflable (3, 3a, 3b, 3c) est traversé sur toute sa longueur par un tube (1, 1a, 1b, 1c) débouchant au delà de celui-ci et qui s'étend sur toute la longueur de la conduite (4) d'insufflation d'air, ce tube étant destiné à assurer une perfusion de sang, ou de liquide physiologique, au delà du ballonnet de dilatation (3, 3a, 3b, 3c) lors de son gonflement,

   - l'extrémité de ce tube (1, 1a, 1b, 1c), qui est opposée à ce ballonnet (3, 3a, 3b, 3c) est raccordée à une pompe de perfusion (11) dont le fonctionnement est asservi à un appareil de commande (17) susceptible d'assurer la synchronisation de son fonctionnement à l'électrocardiogramme du malade à traiter afin d'assurer, en avant du ballonnet de dilatation (3, 3a, 3b, 3c), une perfusion de sang, ou de liquide physiologique transporteur d'oxygène, suivant un débit variable correspondant aux phases des diastoles et des systoles physiologiques du rythme cardiaque de ce malade,

   - la tige de guidage (5, 5a, 5b, 5c) étant montée sur le tube de perfusion (1, 1a, 1b, 1c), ou sur le ballonnet gonflable (3, 3a, 3b, 3c), de façon à ne pas entraver la circulation de liquide à travers ce tube et à la sortie de celui-ci,

2. Appareillage selon la revendication 1, caractérisé en ce que l'appareil de commande (17) de la pompe de perfusion (11) comporte plusieurs dispositifs distincts de commande (18a, 18b, 18c, 18d) aptes à faire fonctionner cette pompe suivant les caractéristiques particulières de la courbe de débit, pendant les phases de diastoles et de systoles, qui est propre à l'une ou l'autre des artères susceptibles d'être traitées -par exemple artère interventriculaire antérieure (IVA), artère circonflexe, artère coronaire droite, artères périphériques-, un organe de commande (20) permettant de mettre en service l'un ou l'autre de ces dispositifs suivant le cas, et l'agencement de l'appareil de commande étant tel que le fonctionnement du dispositif mis en service est asservi à l'électrocardiogramme du malade, en ce qui concerne le moment de départ des phases de diastoles et de systoles.

3. Appareillage selon la revendication 1 ou 2, caractérisé en ce que la tige de guidage (5c) étant destinée à être retirée du cathéter (1c) après mise en place du ballonnet de dilatation (3c) à l'endroit voulu, l'extrémité distale (2c) de ce cathéter comporte plusieurs orifices de sortie (34c) sur sa périphérie pour éviter un effet de jet du sang, ou autre liquide perfusé, à la sortie de l'orifice terminal (7c) de ce cathéter.

4. Appareillage selon l'une des revendications 1 et 2, caractérisé en ce que la tige de guidage (5b) du cathéter (1b) est disposée en dehors de celui-ci en étant simplement engagée à travers le ballonnet gonflable (3b) et l'extrémité distale (2b) du cathéter comporte plusieurs orifices de sortie (34b) sur sa périphérie pour éviter un effet de jet du sang, ou autre liquide perfusé, à la sortie de l'orifice terminal (7b) de ce cathéter.

5. Appareillage selon l'une des revendications 3 et 4, caractérisé en ce que la somme des surfaces des orifices de sortie (34b ou 34c) prévus sur la périphérie de l'extrémité distale (2b ou 2c) du cathéter (1b ou 1c) est supérieure à la section de l'orifice terminal (7b ou 7c) de celui-ci, pour briser la force du jet sortant par l'orifice terminal du cathéter.

6. Appareillage selon l'une des revendications 1 et 2, caractérisé en ce que la tige de guidage (5a) du cathéter (1a) est engagée dans l'extrémité libre (2a) de celui-ci, au delà de la position du ballonnet gonflable (3a), après avoir été disposé à l'extérieur de ce cathéter, et en ce qu'en amont du point d'engagement de cette tige de guidage (5b) dans l'extrémité correspondante du cathéter, cette extrémité comporte des orifices (34) pour la sortie du sang ou liquide physiologique à perfuser.

7. Appareillage selon l'une des revendications 1 et 2, caractérisé en ce que la tige de guidage (5) du cathéter (1) présente une section de surface nettement plus faible que l'espace intérieur de ce cathéter, afin de laisser un passage libre pour le liquide à perfuser, l'extrémité libre (6) de cette tige de guidage pouvant avoir, au-delà de l'orifice terminal (7) du cathéter, une section plus importante, mais permettant néanmoins un coulissement à l'intérieur du cathéter.

**Claims**

1. Apparatus for performing prolonged angioplasty of a coronary artery or peripheral artery, consisting of a catheter comprising an inflatable dilation balloon fixed to the end of an air injection duct associated with a flexible guide rod, chatacterized in that:
- a tube (1, 1a, 1b, 1c) passes throughout the length of the inflatable balloon (3, 3a, 3b, 3c) and opens out beyond it, extending over the entire length of the air injection duct (4), the tube being intended to provide for perfusion of blood or physiological liquid beyond the dilation balloon (3, 3a, 3b, 3c) when the latter is inflated,
- the opposite end of the tube (1, 1a, 1b, 1c) from the balloon (3, 3a, 3b, 3c) is connected to a perfusion pump (11), the operation of which is controlled by a controller (17) capable of ensuring the synchronization of its operation with the electrocardiogram of the patient to be treated in order to provide, ahead of the dilation balloon (3, 3a, 3b, 3c), a perfusion of blood or an oxygen-carrying physiological liquid at a flow rate that varies in accordance with the physiological diastoles and systoles of the patient's cardiac rhythm,
- the guide rod (5, 5a, 5b, 5c) being mounted on the perfusion tube (1, 1a, 1b, 1c), or on the dilation balloon (3, 3a, 3b, 3c), so as not to interfere with the flow of liquid through this tube and at the egress from it,

2. Appatatus as in claim 1, chatacterized in that the controller (17) of the perfusion pump (11) comprises several different control mechanisms (18a, 18b, 18c, 18d) capable of causing the pump to operate in accordance with the special characteristics of the flow curve proper to one or other of the arteries liable to be treated, during the diastoles and systoles (for example: the anterior interventricular artery (IVA), the circumflex artery, the dextral coronary artery, and the peripheral arteries), a control organ (20) permitting one or other of these mechanisms to be brought into service depending on the case, and the arrangement of the controller being such that the operation of the mechanism brought into service is controlled by the electrocardiogram of the patient as far as the start of the diastoles and systoles is concerned.

3. Appatatus as in claim 1 or 2, chatacterized in that, since the guide tod (5c) is intended to be withdrawn from the catheter (1c) after the dilation balloon (3c) has been positioned at the desired location, the distal end (2c) of the catheter includes a series of outlet orifices (34c) arranged on its periphery so as to avoid a jet effect of the blood, or other perfused liquid, at the outlet of the terminal orifice (7c) of the catheter.

4. Appatatus as in one of claims 1 and 2, chatacterized in that the guide tod (5b) of the catheter (1b) is arranged outside the latter by being simply engaged through the inflatable balloon (3b), and the distal end (2b) of the catheter includes several outlet orifices (34b) on its periphery so as to avoid a jet effect of the blood, or other perfused liquid, at the outlet of the terminal orifice (7b) of the catheter.

5. Apparatus as in one of claims 3 and 4, characterized in that the total area of the outlet orifices (34b or 34c) provided on the periphery of the distal end (2b or 2c) of the catheter (1b or 1c) is greater than the cross-sectional area of the terminal orifice (7b or 7c) of the latter, so as to break the force of the jet leaving the terminal orifice of the catheter.

6. Appatatus as in one of claims 1 and 2, chatacterized in that the guide tod (5a) of the catheter (1a) is engaged in the free end (2a) of the latter, beyond the position of the inflatable balloon (3a), after having been arranged on the outside of the catheter, and in that, upstream of the point of engagement of this guide rod (5b) in the corresponding end of the catheter, this end includes orifices (34) for the outflow of the blood or physiological liquid to be perfused.

7. Apparatus as in one of claims 1 and 2, characterized in that the guide rod (5) of the catheter (1) displays a cross-sectional area markedly smaller than the internal space of the catheter in order to leave a free passage for the liquid to be perfused, the free end (6) of the guide rod being capable of having a greater cross-sectional area beyond the terminal orifice (7) of the catheter, while nevertheless permitting sliding within the catheter.


**Patentansprüche**

1. Apparat zur Durchführung einer verlängerten Angioplastie einer Herzkranzarterie oder einer peripheren Arterie, mit einem Kateter, der einen aufblasbaren Dilatationsballon am Ende einer Leitung für Aufblasluft und einen flexiblen Führungsstab aufweist, dadurch gekennzeichnet,
   - daß der aufblasbare Ballon (3, 3a, 3b, 3c) auf seiner gesamten Länge von einem Rohr (1, 1a, 1b, 1c) durchsetzt ist, dessen Auslaßöffnung den Ballon überragt und das sich über die gesamte Länge der Leitung (4) für Aufblasluft erstreckt, wobei das Rohr dem Durchfluß von Blut oder einer physiologischen Flüssigkeit über den Dilatationsballon hinaus bei seinem Aufblasen dient,
   - daß das dem Ballon (3, 3a, 3b, 3c) abgekehrte Ende des Rohres (1, 1a, 1b, 1c) mit einer Perfusionspumpe (11) in Verbindung steht, deren Funktion durch eine Steuereinrichtung (17) synchron zum Elektrokardiogramm des zu behandelnden Patienten gesteuert ist, um über den Dilatationsballon (3, 3a, 3b, 3c) hinaus einen Durchfluß von Blut oder einer physiologischen Flüssigkeit als Sauerstoffträger mit variabler Menge entsprechend den Phasen der physiologischen Diastolen und der Systolen des Herzrhythmusses des Patienten sicherzustellen,
   - und daß der Führungsstab (5, 5a, 5b, 5c) an dem Rohr (1, 1a, 1b, 1c) oder an dem aufblasbaren Ballon (3, 3a, 3b, 3c) so angeordnet ist, daß er die Zirkulation der Flüssigkeit durch das Rohr bis zu seiner Auslaßöffnung nicht behindert.

2. Apparat nach Anspruch 1, dadurch gekennzeichnet, daß die Steuereinrichtung (17) der Perfusionspumpe (11) mehrere bestimmte Steuerkreise (18a, 18b, 18c, 18d) zum Ansteuern dieser Pumpe entsprechend den charakteristischen Besonderheiten der Belastungskurve während der Phasen der Diastolen und der Systolen aufweist, die zum Behandeln der einen oder anderen Arterie geeignet sind, z. B. der Herzkammerschlagader (IVA), der Circonflexarterie, der rechten Herzschlagader, periphärer Arterien, daß die Steuereinrichtung (17) ein Stellorgan (20) zum Einschalten des einen oder anderen der Steuerkreise entsprechend dem gegebenen Fall aufweist, und daß die Steuereinrichtung so ausgebildet ist, daß die Funktion des eingeschalteten Steuerkreises vom Elektrokardiogram des Patienten entsprechend dem Phasenübergang zwischen Diastolen und Systolen gesteuert wird.

3. Apparat nach Anspruch 1 oder 2, dadurch gekennzeichnet, daß der Führungsstab (5c) nach der Plazierung des Dilatationsballons (3c) an der gewünschten Stelle gegenüber dem Katheter (1c) zurückziehbar ausgebildet ist, wobei das distale Ende (2c) des Katheters mehrere über den Umfang verteilte Auslaßöffnungen (34c) zur Vermeidung einer düsenartigen Ausströmung des Bluts oder einer anderen Perfusionsflüssigkeit aus dem endständigen Auslaß (7c) des Katheters aufweist.

4. Apparat nach einem der Ansprüche 1 und 2, dadurch gekennzeichnet, daß der Führungsstab (5c) außerhalb des Katheters (1b) angeordnet ist und den aufblasbaren Ballon (3b) durchsetzt, und daß das distale Ende (2b) des Katheters mehrere über den Umfang verteilte Auslaßöffnungen (34b) zur Verweidung einer düsenartigen Ausströmung des Bluts oder einer anderen Perfusionsflüssigkeit aus dem endständigen Auslaß (7b) des Katheters aufweist.

5. Apparat nach einem der Ansprüche 3 und 4, dadurch gekennzeichnet, daß die Summe der Flächen der über den Umfang verteilten Auslaßöffnungen (34b oder 34c) an distalen Ende (2b oder 2c) des Katheters (1b oder 1c) zur Verminderung der Kraft der düsenartigen Ausströmung durch den endständigen Auslaß des Katheters größer als die Querschnittsfläche des endständigen Auslasses (7b oder 7c) ist.

6. Apparat nach einem der Ansprüche 1 und 2, dadurch gekennzeichnet, daß der Führungsstab (5a) des Katheters (1a) in freien Endbereichen (2a) des Katheters angreift, und zwar über die Position des aufblasbaren Ballons (3a) hinaus, während er ansonsten außerhalb des Katheders angeordnet ist, und daß dieser freie Endbereich stromauf zum Angriffspunkt des Führungsstabs (5a) am Endbereich des Katheters Auslaßöffnungen (34) für Blut oder eine physiologische Perfusionsflüssigkeit aufweist.

7. Apparat nach einem der Ansprüche 1 und 2, dadurch gekennzeichnet, daß der Führungsstab (5) des Katheters (1) einen Abschnitt mit einem wesentlich kleineren Querschnitt als der Innenraum des Katheters aufweist, damit ein Durchfluß für die Perfusionsflüssigkeit belassen ist, und daß das freie Ende (6) des Führungsstabs über den endständigen Auslaß (7) des Katheters hinaus einen Abschnitt mit größerem Querschnitt aufweist, der aber immer noch ein Gleiten im Innenraum des Katheters ermöglicht.

FIG. 1

FIG. 2

FIG. 3

FIG. 4

FIG. 5

FIG. 6

FIG. 7

FIG. 8

FIG. 9

FIG. 10

FIG. 11

FIG. 12

EP 0 366 535 B1